# EUROPEAN PATENT APPLICATION

(11) **EP 4 464 695 A1**
(43) Date of publication of application: **20.11.2024**
(21) Application number: 22920119.9
(22) Date of filing: 12.01.2022
(51) Int. Cl.: C07D 295/24, C07C 239/18, C07C 251/38, C05C 11/00, C09K 19/14, A01C 21/00

(54) **UREASE INHIBITING COMPOSITION AND USE OF SAME**

(71) Applicant: FERTINAGRO BIOTECH, S.L., 44195 Teruel (ES)
(72) Inventor: ATARES REAL, Sergio, 44195 TERUEL (ES); ROMERO LOPEZ, Joaquin, 44195 TERUEL (ES); CABALLERO MOLADA, Marcos, 44195 TERUEL (ES); FERRER GINES, María, 44195 TERUEL (ES); YANCE CHAVEZ, Tula del Carmen, 44195 TERUEL (ES); SALAET MADORRAN, Ignasi, 44195 TERUEL (ES); NARANJO OLIVERO, Miguel Angel, 44195 TERUEL (ES)
(74) Representative: González López-Menchero, Álvaro Luis
(86) International application number: PCT/ES2022/070008
(87) International publication number: WO 2023/135341

(57) **Abstract**

The invention relates to a urease inhibiting composition containing at least one of the compounds of formulas (I), (II) and (III) or a combination thereof: 1,4-dihydroxy-3,6-diisobutylpiperazine-2,5-dione (I), 2-[(N-hydroxyleucyl)-imino]-4-methylpentanoic acid and its keto-enol tautomer (II), and 2-hydroxyimino-4-methyl-N-(3-methylbutylidene)-N-oxide-pentanamide (III).

## Description

The present invention relates to a urease inhibiting composition and the use thereof for soil fertilisation or to reduce ammonia emission in intensive livestock farms.

More specifically, in a first aspect, the invention relates to a urease inhibiting composition containing at least one of the compounds of formulas (I), (II) and (III) or a combination thereof:

| | |
|---|---|
| 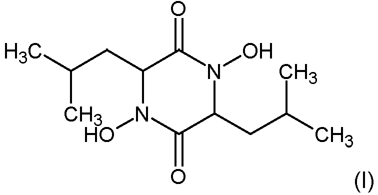 | 1,4-dihydroxy-3,6-disobutylpiperazine-2,5-dione |
| 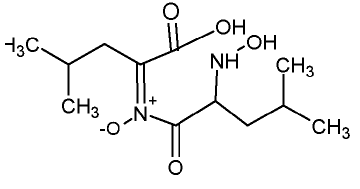 | 2-[(N-hydroxyleucyl)imino]-4-methylpentanoic acid |
| 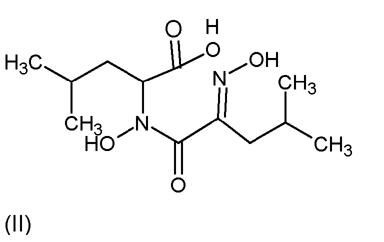 (keto-enol tautomers) | N-hydroxy-N-[2-(hydroxymino)-4-methylpentanoyl]leucine |
| 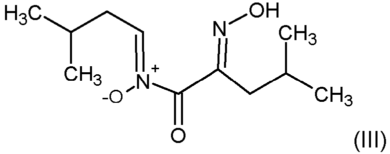 | 2-hydroxymino-4-methyl-N-(3-methylbutylidene)-N-oxide-pentanamide |

In a second aspect, the invention relates to the use of the urease inhibiting composition containing at least one of the compounds of formulas (I), (II) and (III) or a combination thereof to inhibit the action of urease in soils, in slurry from intensive livestock farms, reducing in this case the emission of ammonia, or in the application of urea nitrogen fertilisers, reducing ammonia volatilisation.

Nitrogen is an essential nutrient for crops. Nitrogen, once applied to the ground, undergoes a series of transformations, producing absorption by crops or loss of the nutrient due to different reasons, such as loss due to emissions into the atmosphere of nitrogen oxides and ammonia, loss due to leaching processes or loss due to soil erosion.

For this reason, research has been carried out aimed at developing more efficient fertilisers, such as those developed in patents ES 2 204 307 A1; ES 2/264 386/B1; WO 2001072665 A1; WO 2007/132032; WO 2006 125836 A1.

Urea is one of the most used nitrogen fertilisers in the world mainly due to its high nitrogen content (46%), its low cost, its solubility in water and its easy handling. However, it presents a low yield and a loss in fertiliser effectiveness due to the leaching of nitrogen in nitric form and the losses due to ammoniacal nitrogen volatilisation due to the rapid hydrolysis that urea undergoes when applied to soils:

(NH₂)₂CO + H₂O → CO₂ + 2NH₃

Urea hydrolysis reaction

The ammonia resulting from this hydrolysis reaction enters into equilibrium with the soil moisture, creating ammonium ions:

NH₃ + H₂O → NH₄⁺ + OH⁻

This hydrolysis is catalysed by the enzyme urease, a metalloenzyme the active site of which is composed of a Ni(ll) dimer.

The action of this enzyme is very rapid and causes a high generation of ammonia in very small areas of the soil. Due to the reaction of this NH₃ with the soil, a strong increase in pH occurs in the specific place where the hydrolysis reaction is taking place. For this reason, the NH₄⁺ generation reaction in the soil slows down, accumulating relevant amounts of ammonia (NH₃).

The ammonia released can reach a concentration that is too high in the vicinity of the roots of young plants, causing their intoxication, in addition to its easy release into the environment by volatilisation, with the problems that this may entail.

In this context, any research aimed at developing more effective fertilisers with fewer risks of environmental contamination is of great interest.

In relation to urea hydrolysis inhibitors, a large number of molecules have been developed, both of natural origin (see, for example, Baughman and Wozniak, US5549728; Seiyaku, JP8019595) as synthetic (see, for example, Cheng et al., US5770771; Peacock et al., US5352265; Radel, US4932992).

In the search for urease inhibitors, the selection of these active ingredients is due to the fact that some research suggested that the urease enzyme could be inhibited by molecules derived from hydroxamic acids (Luzia V. Modolo, Aliñe X. de Souza, Lívia P. Horta, Dóbora P. Araujo, Ângelo de Fátima. An overview on the potential of natural products as ureases inhibitors: A review, Journal of Advanced Research (2015) 6, 35-44); Z. Amtul, Atta-ur-Rahman, R.A. Siddiqui and M.I: Choudhary, Chemistry and Mechanism of Urease Inhibition, Current Medicinal Chemistry, 2002, 9, 1323-1348).

Even so, there is literature that reveals the existence of molecules that belong to the group of hydroxamic acids with the capacity to inhibit the urease enzyme *in vitro,* but they are inefficient in the soil and are not capable of reducing the loss of ammoniacal nitrogen from the hydrolysis of urea.

To that end, it would be desirable to have urease inhibitors that are efficient when applied to soils.

According to the first aspect of the invention, this provides a urease inhibiting composition containing at least one of the compounds of formulas (I), (II) and (III) or a combination thereof:

| | |
|---|---|
| 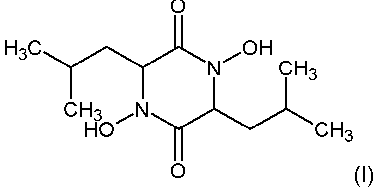 | 1,4-dihydroxy-3,6-disobutylpiperazine-2,5-dione |
| 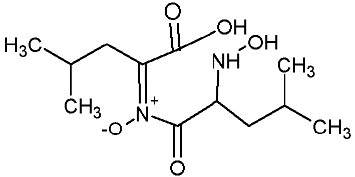 | 2-[(N-hyidroxyleucyl)imino]-4-methylpentanoic acid |
| 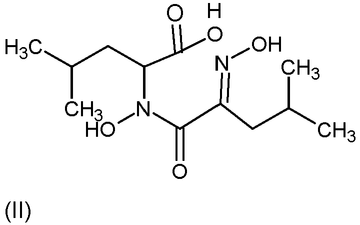 (keto-enol tautomers) | N-hydroxy-N-[2-(hydroxymino)-4-methylpentanoyl]leucine |
| 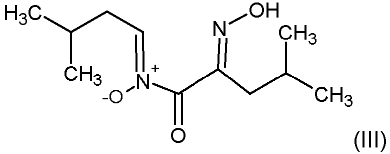 | 2-hydroxymino-4-methyl-N-(3-methylbutylidene)-N-oxide-pentanamide |

in aqueous solution at a final concentration of 1,000 mg of the composition per litre of water.

In one embodiment, the compounds of formulas (I), (II) and (III) described above are present in the urease inhibiting composition of the invention in a concentration equal to or greater than 1 mg/l and up to 50 mg/l.

Preferably, the concentration of the compounds of formulas (I), (II) and (III) described above present in the urease inhibiting composition of the invention is equal to or greater than 5 mg/l.

According to the second aspect, the invention relates to the use of the urease inhibiting composition containing at least one of the compounds of formulas (I), (II) and (III) or a combination thereof to inhibit the action of urease in soils or slurry from intensive livestock farms, reducing in this case the emission of ammonia, or together with urea nitrogen fertilisers, reducing ammonia volatilisation here.

In an embodiment related to this second aspect, in particular when the urease inhibiting composition is used to inhibit urease in soils, the composition is applied at a concentration of between 5 and 100 mg per kilogram of soil. When the urease inhibiting composition is used to inhibit urease in slurries from intensive livestock farms, the composition is applied at a concentration of between 1 and 100 mg per litre of slurry. In the case of the application of the inhibiting composition of the invention together with urea nitrogen fertilisers, preferably the inhibiting composition incorporated in the fertiliser represents between 0.2 and 4 g per kg of urea nitrogen.

The invention is described in more detail below based on exemplary embodiments thereof and with reference to the attached figures, wherein:
- Figure 1:: *in vitro* assay of commercial urease enzyme inhibition "Jack Bean" (from *Canavalia ensiformis,* CAS 9002-13-5, powder) by different amounts of the compounds of formulas (I), (II) and (III);
- Figure 2:: *in vitro* assay of commercial urease enzyme inhibition "Jack Bean" (from *Canavalia ensiformis,* CAS 9002-13-5, powder) by different amounts of compositions of the compounds of formulas (I), (II) and (III);
- Figure 3:: urease inhibition assay in soil samples by different amounts of the compounds of formulas (I), (II) and (III);
- Figure 4:: urease inhibition assay in soil samples by different amounts of compositions of the compounds of formulas (I), (II) and (III);
- Figure 5:: urease inhibition assay in pig slurry samples by different amounts of the compounds of formulas (I), (II) and (III);
- Figure 6:: urease inhibition assay in pig slurry samples by different amounts of compositions of the compounds of formulas (I), (II) and (III);
- Figure 7:: assay on nitrogen loss due to ammonia volatilisation from the hydrolysis of urea in samples of a urea nitrogen fertiliser (Urea Prill) by different amounts of the compounds of formulas (I), (II) and (III); and
- Figure 8:: assay on nitrogen loss due to ammonia volatilisation from the hydrolysis of urea in samples of a urea nitrogen fertiliser (Urea Prill) by different amounts of the compounds of formulas (I), (II) and (III).

### Examples

### Example 1: in vitro assay of commercial urease enzyme inhibition "Jack Bean" (from Canavalia ensiformis, CAS 9002-13-5, powder) by different amounts of the compounds of formulas (I), (II) and (III).

The objective of this assay is to check the capacity that different amounts of the compounds of formulas (I), (II) and (III) have individually to inhibit the action of the commercial urease enzyme "Jack Bean" (SIGMA Type III).

Required material:
- Urea 750 mM in Milli-Q water
- "Jack Bean" urease solution at 0.1 mg/ml in phosphate buffer (1M), with a pH of 7.

Three solutions of the compounds of formulas (I), (II) and (III) are prepared in water at a concentration of 1,000 mg/l each.

The *in vitro* assays are carried out in glass tubes, performing three replicates per sample.

100 µl of phosphate buffer, 100 µl of urea, 20 µl of the urease solution and different doses of the compounds of formulas (I), (II) and (III) are added to each tube for a final concentration in the reaction of 1 mg/l; 5 mg/l; 20 mg/l and 50 mg/l.

All tubes are completed up to 1 ml of final volume with Milli-Q water. The tubes are covered, placed in a controlled environment of temperature (25 °C) and darkness and incubated for 45 minutes. After incubation, 0.1 ml is taken from each tube and added on 0.4 ml of KCI 2M, previously added in new tubes, to stop the enzymatic reaction. Subsequently, the ammonium ion is determined using the Berthelot method.

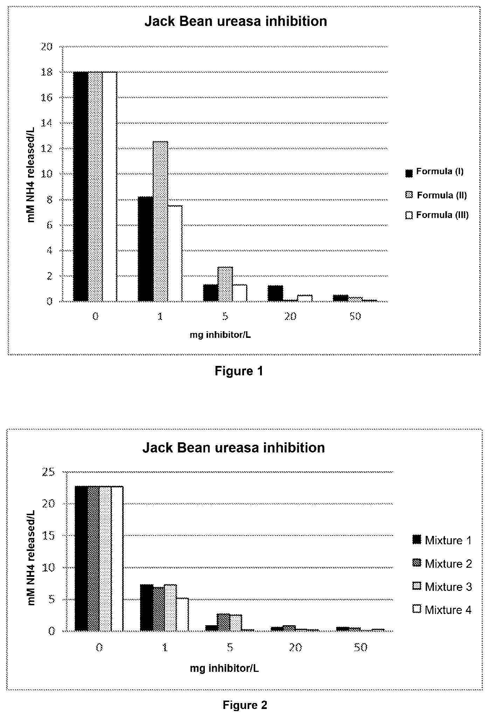

The results of this assay are shown in figure 1.

As derived from figure 1, all three compounds are capable of inhibiting the commercial urease enzyme. This inhibition is greater than 85% when the concentration of each compound is greater than 5 mg/L.

### Example 2: in vitro assay of commercial urease enzyme inhibition "Jack Bean" (from Canavalia ensiformis, CAS 9002-13-5, powder) by different amounts of compositions of the compounds of formulas (I), (II) and (III)

With the objective of assessing the inhibition capacity of mixtures of the compounds of formulas (I), (II) and (III), the following mixtures are prepared:
Mixture 1: 70% compound formula (I) + 30% compound formula (II)
Mixture 2: 50% compound formula (I) + 50% compound formula (II)
Mixture 3: 30% compound formula (II) + 70% compound formula (III)
Mixture 4: 40% compound formula (I) + 20% compound formula (II) + 40% compound formula (III).

Following the protocol of example 1, solutions of these mixtures are prepared at a final concentration of 1,000 mg of mixture/l and the same protocol as in Example 1 is carried out to assess the inhibitory capacity of the commercial urease enzyme Jack Bean.

The results are shown in figure 2.

As can be seen in figure 2, all four combinations are capable of inhibiting *in vitro* urease activity. This inhibition is greater than 70% when the concentration of the combinations is equal to or greater than 1 mg of mixture per litre.

### Example 3: urease inhibition assay in soil samples by different amounts of the compounds of formulas (I), (II) and (III)

With the aim of evaluating the capacity of the compounds of formulas (I), (II) and (III) to inhibit the urease enzyme in soil samples, solutions of said compounds are prepared in water at a concentration of 1,000 mg/l.

Soil inhibition assays are carried out on basic soil samples having a sandy loam texture sieved at 4 mm. For each condition, 1 g of soil sample is taken and 400 µl of water is added for the control sample (NON-inhibiting) and in the inhibition reaction different amounts of inhibiting solution are added to achieve final concentrations of 1, 5, 25, 100 mg of inhibiting compound per kg of soil, completing the remaining volume to 400 µl with water. Samples are incubated at 25°C for 2 hours. After this time, 2.5 ml of 75 mM borate buffer at pH 10 are added and incubated under stirring at room temperature until the sample is as homogeneous as possible, about 15 minutes approximately. 250 µl of the prepared suspension are taken for each of the technical triplicates and 100 µl of substrate and 100 µl of buffer are added for the blank samples (in duplicate). Furthermore, a substrate control is carried out (also in duplicate) where 250 µl of borate buffer and 100 µl of the substrate (urea) are placed.

All samples are incubated at 32°C for 2 hours stirring at 120 rpm. After reaction time, 1 ml of KCI-HCI is added in order to stop the reaction, is incubated with stirring for 15 minutes at room temperature. The tubes are centrifuged for 1 minute at maximum speed and ammonia quantification is carried out using the Berthelot method.

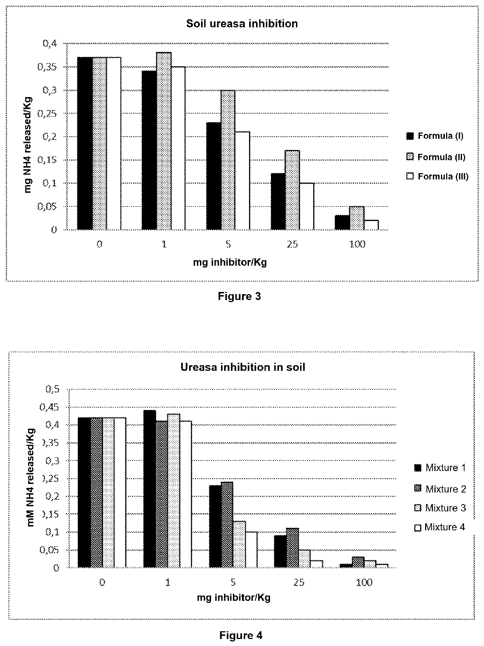

The results are shown in figure 3.

As can be seen in figure 3, all three compounds are capable of inhibiting urease activity in soils. This inhibition is close to 60% when the concentration of the compounds is equal to 25 mg/Kg soil.

### Example 4: urease inhibition assay in soil samples by different amounts of compositions of the compounds of formulas (I), (II) and (III)

With the objective of assessing the urease inhibition capacity in soils of compositions of the compounds of formulas (I), (II) and (III), the following mixtures are prepared:
Mixture 1: 70% compound of formula (I) + 30% compound of formula (II)
Mixture 2: 50% compound of formula (I) + 50% compound of formula (II)
Mixture 3: 30% compound of formula (II) + 70% compound of formula (III)
Mixture 4: 40% compound of formula (I) + 20% compound of formula (II) + 40% compound of formula (III).

Following the protocol of example 3, solutions of these mixtures are prepared at a final concentration of 1,000 mg of mixture/l.

The same protocol as in Example 3 is carried out to assess the inhibition capacity of soil urease enzymes.

The results are shown in figure 4.

As can be seen in figure 4, combinations of different compounds have the capacity to inhibit soil urease activity. Furthermore, combinations 3 and 4 seem to have a cumulative effect, being more efficient than the other two mixtures or the different components separately. In these two combinations we can see inhibitions of 75% or greater at concentrations of 5 mg of mixture/Kg soil.

### Example 5: urease inhibition assay in pig slurry samples by different amounts of the compounds of formulas (I), (II) and (III)

With the aim of evaluating the capacity of the compounds of formulas (I), (II) and (III) to inhibit the urease enzyme in pig slurry samples, solutions of said compounds are prepared separately in water at a concentration of 1,000 mg/l.

Different amounts of the solutions are applied to the slurry to achieve a final concentration of each of the compounds of 1, 5, 25 and 100 mg of compound per litre of slurry.

The basal amount of ammonium in the slurry is high and, for this reason, to assess the urease activity, the slurry with or without the incorporated compounds must be diluted 50 times. To do this, the following protocol is followed with the different slurries to which the different amounts of compounds of formulas (I), (II) and (III) have been added:

| | Basal | Sample |
|---|---|---|
| 1M phosphate buffer | 50 µl | 50 µl |
| Water | 930 µl | 880 µl |
| Slurry (with and without inhibitors) | 20 µl | 20 µl |
| Urea | - | 50 µl |

Once the samples are prepared, the different tubes are incubated for 16 hours at 25 °C. After Incubation, the following steps are followed: taking 200 µl and adding 800 µl of KCI, incubating for 15 minutes, centrifuging at maximum speed, taking 100 µl and placing in a 2 ml Eppendorf, adding 900 µl of water and proceeding with the ammonium quantification protocol according to the Berthelot method.

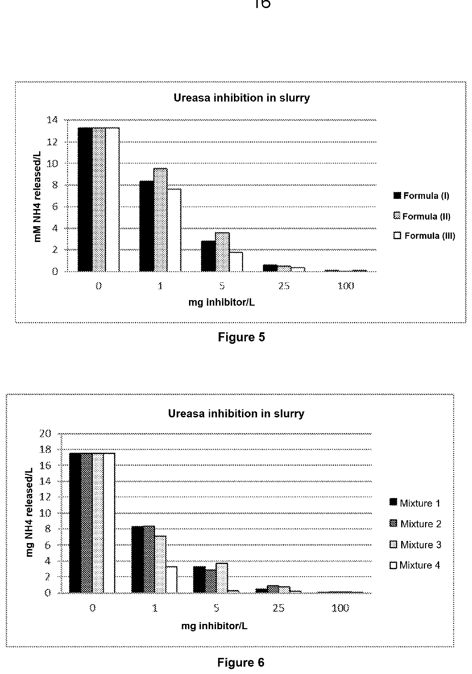

The results are shown in figure 5.

As can be seen in figure 5, incorporating an amount of 5 mg/l of any of the three substances into pig slurry results in a reduction of more than 70% of urease activity.

Example 6: Urease inhibition assay in pig slurry samples by different amounts of compositions of the compounds of formulas (I), (II) and (III)

With the objective of assessing the urease inhibition capacity in slurry samples of compositions of the compounds of formulas (I), (II) and (III), the following mixtures are prepared:
Mixture 1: 70% compound of formula (I) + 30% compound of formula (II)
Mixture 2: 50% compound of formula (I) + 50% compound of formula (II)
Mixture 3: 30% compound of formula (II) + 70% compound of formula (III)
Mixture 4: 40% compound of formula (I) + 20% compound of formula (II) + 40% compound of formula (III).

Different amounts of the mixtures are applied to the slurry to achieve a final concentration of each of 1, 5, 25 and 100 mg of mixture per litre of slurry.

Subsequently, the same protocol as in example 5 is followed to assess the urease activity of the slurry, as well as the inhibiting capacity of the different mixtures.

The results are shown in figure 6.

As can be seen in figure 6, mixtures are more effective than using the substances individually, especially mixture 4, which is capable of reducing the urease activity of slurry by 85% just by applying 1 mg of mixture per litre of slurry.

### Example 7: assay on nitrogen loss due to ammonia volatilisation from the hydrolysis of urea in samples of a urea nitrogen fertiliser (Urea Prill) by different amounts of the compounds of formulas (I), (II) and (III).

With the objective of assessing the impact on nitrogen loss due to ammonia volatilisation from the hydrolysis of urea, the following assay is carried out.

A solution of dimethyl sulphoxide and monopropylene glycol is prepared in a 30/70 ratio. Using this solution, the different compounds of formulas (I), (II) or (III) are incorporated at a rate of 100 g/l.

The solutions are applied to Urea Prill such that the active ingredient remains at a concentration of 0.2, 0.5, 1.0, 2.0 and 4.0 g of compound of formula (I), (II) or (III) per kg of urea nitrogen.

Soil volatilisation assays are carried out on basic soil samples having a sandy loam texture sieved at 4 mm. For each condition, 50 grams of soil sample are taken and placed in a hermetically sealed container. A pad with 2 ml of a mixture of phosphoric acid and glycerine (20/80) is placed on the lid of the container. An amount equivalent to the application of 150 units of nitrogen per hectare is applied to each container and it is closed.

After 7 days, the lid is removed and the pad is recovered by diluting same in 20 ml of water and the amount of ammonia collected in the acid trap (pad with the mixture of phosphoric acid and glycerine) is assessed.

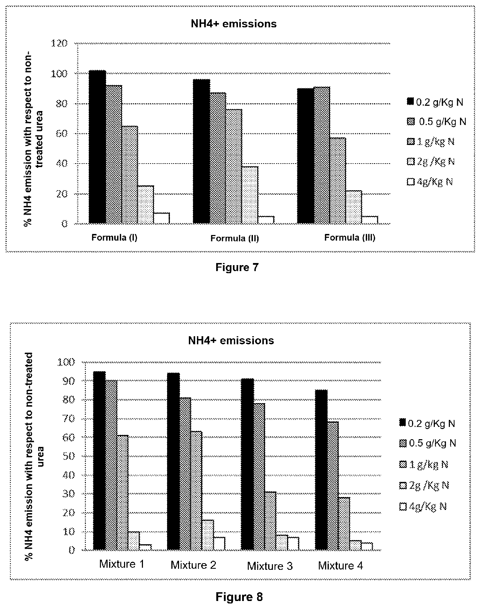

Taking into account the maximum emissions that occur in the application of urea without any treatment, figure 7 shows the values of total ammonium emissions 7 days after the application of treated urea to the soil with respect to the emissions of untreated urea.

As can be seen in figure 7, the use of an amount greater than 1 gram of any of the 3 substances per kg of urea nitrogen causes a reduction in ammonia volatilisation greater than 20%.

### Example 8: assay on nitrogen loss due to ammonia volatilisation from the hydrolysis of urea in samples of a urea nitrogen fertiliser (Urea Prill) by different amounts of the compounds of formulas (I), (II) and (III).

In order to assess the effectiveness of different compositions of the compounds of formulas (I), (II) and (III) on the ammonia volatilisation from the hydrolysis of urea, the following mixtures are prepared:
Mixture 1: 70% compound of formula (I) + 30% compound of formula (II)
Mixture 2: 50% compound of formula (I) + 50% compound of formula (II)
Mixture 3: 30% compound of formula (II) + 70% compound of formula (III)
Mixture 4: 40% compound of formula (I) + 20% compound of formula (II) + 40% compound of formula (III).

A solution of dimethyl sulphoxide and monopropylene glycol is prepared in a 30/70 ratio. The different mixtures 1, 2, 3 and 4 are incorporated to the previous solution at a rate of 100 g/l.

The solutions are applied to Urea Prill such that the sum of the compounds of formulas (I), (II) and (III) is at a concentration of 0.2, 0.5, 1.0, 2.0 and 4.0 g of each compound per kg of urea nitrogen.

The results are shown in figure 8.

As can be seen in figure 8, using an amount greater than 1 gram of any of the 4 mixtures per Kg of urea nitrogen results in a 40% reduction in ammonia volatilisation, this value reaches 70% in the case of mixtures 3 and 4.

## Claims

1. A urease inhibiting composition containing at least one of the compounds of formulas (I), (II) and (III) or a combination thereof:
| | |
|---|---|
| 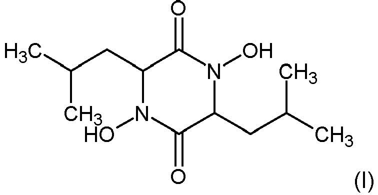 | 1,4-dihydroxy-3,6-diisobutylpiperazine-2,5-dione |
| 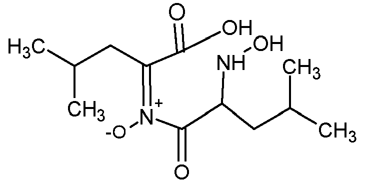 | 2-[(N-hydroxyleucyl)imino]-4-methylpentanoic acid |
| 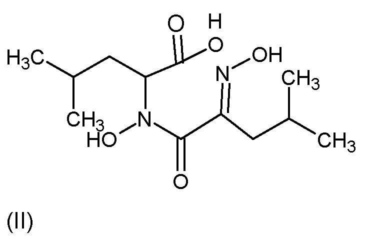 (keto-enol tautomers) | N-hydroxy-N-[2-(hydroxymino)-4-methylpentanoyl]leucine |
| 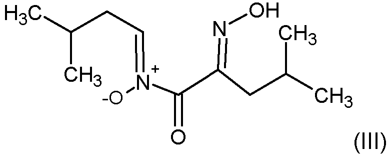 | 2-hydroxymino-4-methyl-N-(3-methylbutylidene)-N-oxide-pentanamide |

2. The urease inhibiting composition according to claim 1, **characterised in that** the compounds of formulas (I), (II) and (III) are present in the urease inhibiting composition in a concentration equal to or greater than 1 mg/l and up to 50 mg/l.

3. The urease inhibiting composition according to claim 2, **characterised in that** the concentration of the compounds of formulas (I), (II) and (III) present in the urease inhibiting composition of the invention is equal to or greater than 5 mg/l.

4. A use of the urease inhibiting composition containing at least one of the compounds of formulas (I), (II) and (III) or a combination thereof according to claim 1 to inhibit the action of urease in soils.

5. A use of the urease inhibiting composition containing at least one of the compounds of formulas (I), (II) and (III) or a combination thereof according to claim 1 to inhibit the action of urease in slurry.

6. A use of the urease inhibiting composition containing at least one of the compounds of formulas (I), (II) and (III) or a combination thereof according to claim 1 together with urea nitrogen fertilisers.

7. A use of the urease inhibiting composition according to claim 4, **characterised by** a concentration of between 5 and 100 mg per kilogram of soil.

8. A use of the urease inhibiting composition according to claim 5, **characterised by** a concentration of between 1 and 100 mg per litre of slurry.

9. A use of the urease inhibiting composition according to claim 6, **characterised by** being incorporated into the fertiliser representing between 0.2 and 4.0 g per kg of urea nitrogen.
